# EUROPEAN PATENT SPECIFICATION

(11) **EP 3 441 457 B1**
(45) Date of publication and mention of the grant of the patent: **06.01.2021**
(21) Application number: 17898958.8
(22) Date of filing: 01.03.2017
(51) Int. Cl.: C12N 5/04, A01H 4/00, A01H 3/04, A01N 61/00

(54) **METHOD FOR INCREASING CONTENT OF MOGROSIDE V IN SIRAITIA GROSVENORII SUSPENDED CELLS**
VERFAHREN ZUR ERHÖHUNG DES GEHALTS AN MOGROSID V IN SIRAITIA GROSVENORI ZELLSUSPENSIONEN
PROCÉDÉ POUR AUGMENTER LA TENEUR EN MOGROSIDE V DANS DES CELLULES DE SIRAITIA GROSVENORII

(43) Date of publication of application: 13.02.2019
(73) Proprietor: Guilin Layn Natural Ingredients Corp., Guilin, Guangxi 541199 (CN)
(72) Inventor: SONG, Yunfei, Guilin Guangxi 541199 (CN); YANG, Wenguo, Guilin Guangxi 541199 (CN); LI, Yuanyuan, Guilin Guangxi 541199 (CN); GUO, Meijin, Guilin Guangxi 541199 (CN); LI, Jiarui, Guilin Guangxi 541199 (CN); WANG, Zejian, Guilin Guangxi 541199 (CN); ZHUANG, Yingping, Guilin Guangxi 541199 (CN)
(74) Representative: Ström & Gulliksson AB
(86) International application number: PCT/CN2017/075348
(87) International publication number: WO 2018/157335

(56) References cited:
- WO-A1-2014/086842
- WO-A1-2015/082012
- CN-A- 1 650 696
- CN-A- 1 683 387
- CN-A- 101 007 042
- CN-A- 101 801 177
- CN-A- 104 059 959
- CN-A- 104 431 841
- KAILUN ZHANG ET AL: "Methyl jasmonate-induced accumulation of metabolites and transcriptional responses involved in triterpene biosynthesis in Siraitia grosvenorii fruit at different growing stages", ACTA SOCIETATIS BOTANICORUM POLONIAE, vol. 85, no. 3, 30 September 2016 (2016-09-30), page 3503, XP055634664, PL ISSN: 0001-6977, DOI: 10.5586/asbp.3503
- ZHANG, KAILUN: "The Effects of Methyl Jasmonate on the biosynthetic Pathway of Mogrosides and the Preliminary Screening of Bhlh Transcription Factors in Siraitia Grosvenorii", China Master S Theses Full - Text Database Agriculture Science, 15 February 2017 (2017-02-15), pages 1-71, XP009515622,
- ZENG, WENWEN: "Establishment of Genetic Transformation System for Siraitia Grosvenorii And Transformation Research of Cs Gene", Master Thesis, 15 March 2016 (2016-03-15), pages 1-74, XP009515623,
- HUANG, ZHUANGJIA et al.: "Advances in Studies on Key Post-Modification Enzymes in Triterpenoid Saponins Biosynthesis", Acta Botanica Boreali-Occidentalia Sinica, vol. 34, no. 10, 31 October 2014 (2014-10-31), pages 2137-2144, XP9515278,

## Description

### FIELD OF THE INVENTION

The present invention relates to the field of biotechnology, in particular to a method for increasing the content of mogroside V in *Siraitia grosvenorii* suspended cells.

### BACKGROUND OF THE INVENTION

Mogroside V, also known as mogroside, is the main sweet component of *Siraitia grosvenori,* and its sweetness is 350 times that of sucrose. Seven monomer components have been identified so far. Among them, mogroside V and Siamenoside I have the strongest sweetness. The mogroside V has the functions of clearing heat and moistening lung, relieving sore-throat to restore voice, relaxing bowel and other medicinal effects; besides, the mogroside V is also popular with people for its unique and pure taste. It is the most suitable sweetener and health care product for people with obesity, hypertension and diabetes, and other special people. It has a wide range of application value. At present, the method of obtaining the mogroside V is mainly based on the natural *Siraitia grosvenorii* as the raw material, which is prepared into the mogroside V by heating and other methods. However, the content of intrinsic mogroside V components in the natural *Siraitia grosvenorii* is extremely low. The prior art has a low utilization rate for the *Siraitia grosvenorii* and a low extraction rate for the mogroside V, resulting in a great waste of raw materials, not meeting the growing market demand.

The production of target secondary metabolites by the plant cell culture technology is one of the effective means to increase the content of target components in natural plants. Cell suspension cultures have the characteristics of rapid propagation, large scale of culture, and providing a large number of uniform plant cell cultures. Therefore, the culture of the mogroside V by the plant cell culture technology can effectively increase the content of the mogroside V and reduce the production cost, which is conducive to the further marketization of mogroside V. For example, Chinese Patent Publication No. CN102174463B of Reference 1 disclosed "*A Method for Culturing a Siraitia Grosvenorii Callus Cell Suspension System",* by which the *Siraitia grosvenorii* suspended cells were obtained in which total glucoside of *Siraitia grosvenorii* and mogroside V accounted for 8.11% and 5.77% of the total weight of the cells, respectively. **However, the culture cycle of the method was as long as 21 days,** which increased the difficulty and cost of operation and maintenance, and the obtained content was still far from satisfying the market demand for the mogroside V.

In the process of plant secondary metabolism, an inducer, as a special biochemical molecule, can rapidly, specifically and selectively induce the expression of certain specific genes, thereby increasing the activity of related enzymes in metabolic pathways, which in turn promotes or inhibits the production of secondary products regulated by the enzymes. A fungal inducer, as a definitive chemical signal from a microbial molecule, can stimulate a plant cell to make a rapid defense response, thereby highly specifically and selectively activating the expression of a specific plant gene, which in turn activates the specific secondary metabolic pathways and promotes the synthesis of secondary metabolites. For example, it was recorded in the Chinese document of Reference 2 *"*Establishment of Siraitia Grosvenorii Cell Culture System and Regulation of Mogroside V Formation" (Chen Langui, Guangxi Normal University, 2010) that the addition of a component (protein + polysaccharide) in the Aspergillus niger crude extract having a total sugar concentration of 300 mg/L increased the total amount of mogroside V by 246.72% compared with the control group; the addition of 500 mg/L of phenylalanine significantly promoted the synthesis of mogroside V, with the total amount of mogroside V increased by 260.38% compared with the control group. **Although this method increased the content of mogroside V, it also increased the secretion synthesis of bitter and astringent components,** and increased the difficulty and cost of isolation and purification of the mogroside V in the subsequent preparation process.

Yeast is a commonly used inducer in plant cell culture and can induce or promote the formation of secondary metabolites. Studies showed that the addition of a yeast inducer doubled the growth of Cryptotanshinone in Ti-transformed Salvia miltiorrhiza cell culture. The yeast also increased the content of Plumbagin in Plumbago roseal by 3 times. However, different systems have different induction effects. Methyl jasmonate (MeJA) is a non-biological inducing factor that stimulates the accumulation of secondary metabolites. Addition of methyl jasmonate to different plant suspension culture systems can induce the accumulation of secondary metabolites in cells. After 200 µmol/L of methyl jasmonate had been used to treat the suspension culture cells of *Maackia amurensis* Rupr. et Maxim for 9 days, the content of isoflavone was increased to 417.18% of that of the control of the same period; when 100 µmol/L of methyl jasmonate was used to treat *Taxus chinensis* suspended cells, the content of paclitaxel was increased by 10 times. However, the addition of methyl jasmonate to some plant suspension culture systems was also easy to induce allergic reactions in suspended cells or promote the release of phenolic compounds, resulting in browning of suspended cells. When 1.0 mg/L of methyl jasmonate was used to treat pueraria suspended cells, the content of puerarin in the cell culture was increased, but the cells were significantly browned. At present, there is no report on the treatment of *Siraitia grosvenorii* suspended cell culture system with yeast or methyl jasmonate.

### CONTENTS OF THE INVENTION

In order to overcome the deficiencies in the prior art, a purpose of the present invention is to provide a method for increasing the content of mogroside V in *Siraitia grosvenorii* suspended cells. The method not only shortens the production and culture cycle of the *Siraitia grosvenorii* suspended cells and reduces the operation and maintenance costs in the production process, but also effectively increases the content of mogroside V in the *Siraitia grosvenorii* suspended cells, and simultaneously reduces the synthesis and secretion of bitter and astringent components.

The inventor of the present invention has found that in the process of culturing the mature *Siraitia grosvenorii* suspended cells in the primary *Siraitia grosvenorii* suspended cell system, the addition of an appropriate amount of a yeast inducer and methyl jasmonate can effectively increase the content of mogroside V in the *Siraitia grosvenorii* suspended cells and accelerate its synthesis, while reducing the synthesis and secretion of **mogroside II.**

The purpose of the present invention can be achieved by the following technical solution:
**The present invention provides a method for increasing the content of mogroside V in *Siraitia grosvenorii*** suspended cells. **The method is characterized in that** a yeast inducer and methyl jasmonate are added during culture of mature *Siraitia grosvenorii* suspended cells in a primary *Siraitia grosvenorii* suspended cell system.

**Preferably,** during the culture of the mature *Siraitia grosvenorii* suspended cells in the primary *Siraitia grosvenorii* suspended cell system, 10-50 mg/L of the yeast inducer and 50-100 µmol/L of methyl jasmonate are added on days 5 to 7, 100-200 mg/L of the yeast inducer and 120-260 µmοl/L of methyl jasmonate are added on days 9 to 11, and the mature *Siraitia grosvenorii* suspended cells are harvested on days 17 to 25.

**Preferably,** during the culture of the mature *Siraitia grosvenorii* suspended cells in the primary *Siraitia grosvenorii* suspended cell system, 50 mg/L of the yeast inducer and 100 µmol/L of methyl jasmonate are added on day 7, 180 mg/L of the yeast inducer and 220 µmol/L of methyl jasmonate are added on day 11, and the mature *Siraitia grosvenorii* suspended cells are harvested on day 19.

**Preferably,** the primary *Siraitia grosvenorii* suspended cell system is obtained by the following steps:
(1) Taking mature *Siraitia grosvenorii* embryos, subculturing on an MS semi-solid medium containing 30 g/L of sucrose, 1.0 mg/L of 6-benzylamino adenine, 0.5 mg/L of naphthaleneacetic acid, 100 mg/L of inositol and 4.6 g/L of agar in a pH range of 5.5-6.0, and selecting fresh embryogenic calli that are successively subcultured 3-5 times, grow vigorously, and have loose texture and a stable and uniform state; and
(2) the fresh embryogenic calli obtained in the step (1) are transferred at an inoculum amount of 100 g/L to an MS liquid medium containing 30 g/L of sucrose, 1.0 mg/L of 6-benzylamino adenine, 0.5 mg/L of naphthaleneacetic acid and 100 mg/L of inositol in a pH range of 5.5-6.5, and cultured in a shaker at a rotation speed of 150 r/min, a culture temperature of (25±2)°C, a light intensity of 4000 lux and an illumination time of 12 h/d, so that the primary *Siraitia grosvenorii* suspended cell system is obtained.

Preferably, the subculture is performed in the step (1) for 4 times.

**The present invention overcomes the following technical problems:** After treatment of the *Siraitia grosvenorii* suspended cells in the logarithmic growing period with methyl jasmonate alone, **the amount of synthesis of mogroside V per unit time increased,** but the cell browning phenomenon was obvious, **and the total content decreased compared with the normal culture group.** Addition of a yeast inducer alone had no significant effect on the total content of mogroside V, but **the synthesis rate of mogroside V decreased.**

After a series of technical optimizations, it was found that the following steps could significantly increase the synthesis rate and total content of mogroside V, significantly reduce the synthesis and secretion of bitter and astringent components, and significantly shorten the cell culture cycle: 10-50 mg/L of the yeast inducer and 50-100 µmol/L of methyl jasmonate were added on days 5 to 7 of the culture of the primary *Siraitia grosvenorii* suspended cell system, 100-200 mg/L of the yeast inducer and 120-260 µmol/L of methyl jasmonate were added on days 9 to 11 of the culture, and mature *Siraitia grosvenorii* suspended cells were harvested on days 17 to 25 of the culture. Specifically, 50 mg/L of the yeast inducer and 100 µmol/L of methyl jasmonate were added on day 7 of the culture, 180 mg/L of the yeast inducer and 220 µmol/L of methyl jasmonate were added on day 11 of the culture, and the mature *Siraitia grosvenorii* suspended cells were harvested on day 19 of the culture, making the mogroside V have the highest synthesis rate and total content, and making the bitter and astringent components **mogroside II** have the least synthesis and secretion.

**The present invention also provides a method for preparing mogroside V.** It is characterized in that the mature *Siraitia grosvenorii* suspended cells prepared by the above method are further extracted and isolated to prepare the mogroside V.

**The present invention has the following advantages compared with the prior art:**
1. According to the present invention, the *Siraitia grosvenorii* embryo is used as a culture raw material, making the differentiation and passage capacity stronger and more stable, favorable for culturing the *Siraitia grosvenorii* calli that have vigorous growth and stable state.
2. The *Siraitia grosvenorii* cell suspension system cultured by the method of the present invention has obviously increased yield of mogroside V, and less synthesized and secreted bitter and astringent impurities.
3. The *Siraitia grosvenorii* cell suspension system cultured by the method of the present invention has the advantages of high cell growth speed, good dispersibility, short culture cycle, and reduced operation and maintenance difficulty, better meeting the requirements of marketized production.
4. The method of the present invention to obtain the mogroside V saves a large amount of land resources, is conducive to the protection of the ecological environment, not only in line with China's economic strategy for sustainable development, but also providing an effective way of solving the source problem of mogroside V.
5. The method of the present invention artificially regulates the production process of *Siraitia grosvenorii* cells and mogroside V, conducive to quality control of products.

### DETAILED DESCRIPTION OF THE EMBODIMENTS

The following examples are intended to illustrate the present invention, but are not intended to limit the scope thereof.

### Example 1

(1) Taking mature *Siraitia grosvenorii* embryos, subculturing on an MS semi-solid medium containing 30 g/L of sucrose, 1.0 mg/L of 6-benzylamino adenine, 0.5 mg/L of naphthaleneacetic acid, 100 mg/L of inositol and 4.6 g/L of agar in a pH range of 5.5-6.0, and selecting fresh embryogenic calli that were successively subcultured for 4 times, grew vigorously, and had loose texture and a stable and uniform state;
(2) the fresh embryogenic calli obtained in the step (1) were transferred at an inoculum amount of 100 g/L to an MS liquid medium containing 30 g/L of sucrose, 1.0 mg/L of 6-benzylamino adenine, 0.5 mg/L of naphthaleneacetic acid and 100 mg/L of inositol in a pH range of 5.5-6.5, and cultured in a shaker at a rotation speed of 150 r/min, a culture temperature of (25±2)°C, a light intensity of 4000 lux and an illumination time of 12 h/d, so that the primary *Siraitia grosvenorii* suspended cell system was obtained;
(3) the mature *Siraitia grosvenorii* suspended cells were cultured in the primary *Siraitia grosvenorii* suspended cell system obtained in the step (2); 50 mg/L of a yeast inducer and 100 µmol/L of methyl jasmonate were added on day 7 of the culture, and 180 mg/L of the yeast inducer and 220 µmol/L of methyl jasmonate were added on day 11; and
(4) on days 1 to 31 of the culture, the *Siraitia grosvenorii* suspended cells were extracted every other day to detect the **content** of mogroside V and **mogroside II.**

### Example 2

(1) Taking mature *Siraitia grosvenorii* embryos, subculturing on an MS semi-solid medium containing 30 g/L of sucrose, 1.0 mg/L of 6-benzylamino adenine, 0.5 mg/L of naphthaleneacetic acid, 100 mg/L of inositol and 4.6 g/L of agar in a pH range of 5.5-6.0, and selecting fresh embryogenic calli that were successively subcultured 3 times, grew vigorously, and had loose texture and a stable and uniform state;
(2) the fresh embryogenic calli obtained in the step (1) were transferred at an inoculum amount of 100 g/L (fresh weight) to an MS liquid medium containing 30 g/L of sucrose, 1.0 mg/L of 6-benzylamino adenine, 0.5 mg/L of naphthaleneacetic acid and 100 mg/L of inositol in a pH range of 5.5-6.5, and cultured in a shaker at a rotation speed of 150 r/min, a culture temperature of (25±2)°C, a light intensity of 4000 lux and an illumination time of 12 h/d, so that the primary *Siraitia grosvenorii* suspended cell system was obtained;
(3) the mature *Siraitia grosvenorii* suspended cells were cultured in the primary *Siraitia grosvenorii* suspended cell system obtained in the step (2); 10 mg/L of a yeast inducer and 50 µmol/L of methyl jasmonate were added on day 5 of the culture, and 100 mg/L of the yeast inducer and 120 µmol/L of methyl jasmonate were added on day 9; and
(4) on days 1 to 31 of the culture, the *Siraitia grosvenorii* suspended cells were extracted every other day to detect the **content** of mogroside V and **mogroside II.**

### Example 3

(1) Taking mature *Siraitia grosvenorii* embryos, subculturing on an MS semi-solid medium containing 30 g/L of sucrose, 1.0 mg/L of 6-benzylamino adenine, 0.5 mg/L of naphthaleneacetic acid, 100 mg/L of inositol and 4.6 g/L of agar in a pH range of 5.5-6.0, and selecting fresh embryogenic calli that were successively subcultured 5 times, grew vigorously, and had loose texture and a stable and uniform state;
(2) the fresh embryogenic calli obtained in the step (1) were transferred at an inoculum amount of 100 g/L (fresh weight) to an MS liquid medium containing 30 g/L of sucrose, 1.0 mg/L of 6-benzylamino adenine, 0.5 mg/L of naphthaleneacetic acid and 100 mg/L of inositol in a pH range of 5.5-6.5, and cultured in a shaker at a rotation speed of 150 r/min, a culture temperature of (25±2)°C, a light intensity of 4000 lux and an illumination time of 12 h/d, so that the primary *Siraitia grosvenorii* suspended cell system was obtained;
(3) the mature *Siraitia grosvenorii* suspended cells were cultured in the primary *Siraitia grosvenorii* suspended cell system obtained in the step (2); 25 mg/L of a yeast inducer and 75 µmol/L of methyl jasmonate were added on day 7 of the culture, and 200 mg/L of the yeast inducer and 260 µmol/L of methyl jasmonate were added on day 11; and
(4) on days 1 to 31 of the culture, the *Siraitia grosvenorii* suspended cells were extracted every other day to detect the **content** of mogroside V and **mogroside II.**

### Example 4

(1) Inoculating with and inducing *Siraitia grosvenorii* stem calli, subculturing on an MS medium containing 2.5% of sucrose, 0.2 mg/L of 6-benzyladenine, 0.04 mg/L of naphthylacetic acid and 4.0 mg/L of agar at a pH of 5.8, and selecting fresh, soft and fragile yellow-white calli that were successively subcultured 3, 4 or 5 times;
(2) the calli obtained in the step (1) were transferred at an inoculum amount of 300 g/l to the MS liquid medium in a sterile triangular flask containing 4.0% of sucrose, 1.0 mg/L of 6-benzylamino adenine, and 0.02 mg/L of naphthalene acetic acid, with the pH of the initial medium at 5.5 to 6.0; after suspension culture in a shaking incubator at 120 r/min in the dark at 25°C for 24 h, the medium was left to stand, and the upper medium together with the single cells and small cell clusters was transferred to a sterile empty triangular flask to proceed with shaking culture;
(3) after 3 days, the same method was used to transfer once again; after 7 days, the suspended cells were filtered through a 40 µm sieve, and inoculated into a fresh medium to proceed with the shaking culture; after reaching a certain volume, the suspended cells were transferred to a large triangular flask to proceed with the shaking culture with the addition of medium in the same way, until a certain amount of uniform and stable cell suspension culture fluid was reached; the cell suspension culture temperature was (25±2)°C, the light intensity was 2000 lux, and the illumination time was 12 h/d; the primary *Siraitia grosvenorii* suspended cell system was thus obtained;
(4) the mature *Siraitia grosvenorii* suspended cells were cultured in the primary *Siraitia grosvenorii* suspended cell system obtained in the step (3); a yeast inducer of 50 mg/L and methyl jasmonate of 100 µmοl/L were added on day 7 of the culture, and the yeast inducer 180 mg/L and methyl jasmonate of 220 µmοl/L were added on day 11; and
(5) on days 1 to 31 of the culture, the *Siraitia grosvenorii* suspended cells were extracted every other day to detect the **content** of mogroside V and **mogroside II.**

### Comparative Example 1

This comparative example was used to evaluate the difference in technical effects between the technical solution of adding an Aspergillus niger inducer and methyl jasmonate and the technical solution of the present invention. The specific steps were as follows:
(1) Taking mature *Siraitia grosvenorii* embryos, subculturing on an MS semi-solid medium containing sucrose of 30 g/L, 6-benzylamino adenine of 1.0 mg/L, naphthaleneacetic acid of 0.5 mg/L, inositol of 100 mg/L and agar of 4.6 g/L in a pH range of 5.5-6.0, and selecting fresh embryogenic calli that were successively subcultured for 4 times, grew vigorously, and had loose texture and a stable and uniform state;
(2) the fresh embryogenic calli obtained in the step (1) were transferred at an inoculum amount of 100 g/L to an MS liquid medium containing sucrose of 30 g/L, 6-benzylamino adenine of 1.0 mg/L, naphthaleneacetic acid of 0.5 mg/L and inositol of 100 mg/L in a pH range of 5.5-6.5, and cultured in a shaker at a rotation speed of 150 r/min, a culture temperature of (25±2)°C, a light intensity of 4000 lux and an illumination time of 12 h/d, so that the primary *Siraitia grosvenorii* suspended cell system was obtained;
(3) the mature *Siraitia grosvenorii* suspended cells were cultured in the primary *Siraitia grosvenorii* suspended cell system obtained in the step (2); an Aspergillus niger inducer of 50 mg/L and methyl jasmonate of 100 µmοl/L were added on day 7 of the culture, and the Aspergillus niger inducer of 180 mg/L and methyl jasmonate of 220 µmol/L were added on day 11; and
(4) on days 1 to 31 of the culture, the *Siraitia grosvenorii* suspended cells were extracted every other day to detect the **content** of mogroside V and **mogroside II.**

### Comparative Example 2

This comparative example was used to evaluate the difference in technical effects between the technical solution of adding phenylalanine and a yeast inducer and the technical solution of the present invention. The specific steps were as follows:
(1) Taking mature *Siraitia grosvenorii* embryos, subculturing on an MS semi-solid medium containing sucrose of 30 g/L, 6-benzylamino adenine of 1.0 mg/L, naphthaleneacetic acid of 0.5 mg/L, inositol of 100 mg/L and agar of 4.6 g/L in a pH range of 5.5-6.0, and selecting fresh embryogenic calli that were successively subcultured for 4 times, grew vigorously, and had loose texture and a stable and uniform state;
(2) the fresh embryogenic calli obtained in the step (1) were transferred at an inoculum amount of 100 g/L to an MS liquid medium containing sucrose of 30 g/L, 6-benzylamino adenine of 1.0 mg/L, naphthaleneacetic acid of 0.5 mg/L and inositol of 100 mg/L in a pH range of 5.5-6.5, and cultured in a shaker at a rotation speed of 150 r/min, a culture temperature of (25±2)°C, a light intensity of 4000 lux and an illumination time of 12 h/d, so that the primary *Siraitia grosvenorii* suspended cell system was obtained;
(3) the mature *Siraitia grosvenorii* suspended cells were cultured in the primary *Siraitia grosvenorii* suspended cell system obtained in the step (2); phenylalanine of 50 mg/L and the yeast inducer of 50 mg/L were added on day 7 of the culture, and phenylalanine of 180 mg/L and the yeast inducer of 180 mg/L were added on day 11 of the culture; and
(4) on days 1 to 31 of the culture, the *Siraitia grosvenorii* suspended cells were extracted every other day to detect the **content** of mogroside V and **mogroside II.**

### Comparative Example 3

This comparative example was used to evaluate the difference in technical effects between the technical solution of adding the yeast inducer alone and the technical solution of the present invention. The specific steps were as follows:
(1) Taking mature *Siraitia grosvenorii* embryos, subculturing on an MS semi-solid medium containing sucrose of 30 g/L, 6-benzylamino adenine of 1.0 mg/L, naphthaleneacetic acid of 0.5 mg/L, inositol of 100 mg/L and agar of 4.6 g/L in a pH range of 5.5-6.0, and selecting fresh embryogenic calli that were successively subcultured for 4 times, grew vigorously, and had loose texture and a stable and uniform state;
(2) the fresh embryogenic calli obtained in the step (1) were transferred at an inoculum amount of 100 g/L to an MS liquid medium containing sucrose of 30 g/L, 6-benzylamino adenine of 1.0 mg/L, naphthaleneacetic acid of 0.5 mg/L and inositol of 100 mg/L in a pH range of 5.5-6.5, and cultured in a shaker at a rotation speed of 150 r/min, a culture temperature of (25±2)°C, a light intensity of 4000 lux and an illumination time of 12 h/d, so that the primary *Siraitia grosvenorii* suspended cell system was obtained;
(3) the mature *Siraitia grosvenorii* suspended cells were cultured in the primary *Siraitia grosvenorii* suspended cell system obtained in the step (2); the yeast inducer of 50 mg/L was added on day 7 of the culture, and the yeast inducer of 180 mg/L was added on day 11; and
(4) on days 1 to 31 of the culture, the *Siraitia grosvenorii* suspended cells were extracted every other day to detect the **content** of mogroside V and **mogroside II.**

### Comparative Example 4

This comparative example was used to evaluate the difference in technical effects between the technical solution of adding methyl jasmonate alone and the technical solution of the present invention. The specific steps were as follows:
(1) Taking mature *Siraitia grosvenorii* embryos, subculturing on an MS semi-solid medium containing sucrose of 30 g/L, 6-benzylamino adenine of 1.0 mg/L, naphthaleneacetic acid of 0.5 mg/L, inositol of 100 mg/L and agar of 4.6 g/L in a pH range of 5.5-6.0, and selecting fresh embryogenic calli that were successively subcultured for 4 times, grew vigorously, and had loose texture and a stable and uniform state;
(2) the fresh embryogenic calli obtained in the step (1) were transferred at an inoculum amount of 100 g/L to an MS liquid medium containing sucrose of 30 g/L, 6-benzylamino adenine of 1.0 mg/L, naphthaleneacetic acid of 0.5 mg/L and inositol of 100 mg/L in a pH range of 5.5-6.5, and cultured in a shaker at a rotation speed of 150 r/min, a culture temperature of (25±2)°C, a light intensity of 4000 lux and an illumination time of 12 h/d, so that the primary *Siraitia grosvenorii* suspended cell system was obtained;
(3) the mature *Siraitia grosvenorii* suspended cells were cultured in the primary *Siraitia grosvenorii* suspended cell system obtained in the step (2); methyl jasmonate of 100 µmol/L was added on day 7 of the culture, and methyl jasmonate of 220 µmol/L was added on day 11; and
(4) on days 1 to 31 of the culture, the *Siraitia grosvenorii* suspended cells were extracted every other day to detect the **content** of mogroside V and **mogroside II.**

### Comparative Example 5

This comparative example was used to evaluate the difference in technical effects between the technical solution disclosed in Reference 1 and the technical solution of the present invention. The specific steps were as follows:
(1) Inoculating with and inducing *Siraitia grosvenorii* stem calli, subculturing on an MS medium containing 2.5% of sucrose, 0.2 mg/L of 6-benzyladenine, 0.04 mg/L of naphthylacetic acid and 4.0 mg/L of agar at a pH of 5.8, and selecting fresh, soft and fragile yellow-white calli that were successively subcultured 3, 4 or 5 times;
(2) the calli obtained in the step (1) were transferred at an inoculum amount of 300 g/l to the MS liquid medium in a sterile triangular flask containing 4.0% of sucrose, 1.0 mg/L of 6-benzylamino adenine, and 0.02 mg/L of naphthalene acetic acid, with the pH of the initial medium at 5.5 to 6.0; after suspension culture in a shaking incubator at 120 r/min in the dark at 25°C for 24 h, the medium was left to stand, and the upper medium together with the single cells and small cell clusters was transferred to a sterile empty triangular flask to proceed with shaking culture;
(3) after 3 days, the same method was used to transfer once again; after 7 days, the suspended cells were filtered through a 40 µm sieve, and inoculated into a fresh medium to proceed with the shaking culture; after reaching a certain volume, the suspended cells were transferred to a large triangular flask to proceed with the shaking culture with the addition of medium in the same way, until a certain amount of uniform and stable cell suspension culture fluid was reached; the cell suspension culture temperature was (25±2)°C, the light intensity was 2000 lux, and the illumination time was 12 h/d; the primary *Siraitia grosvenorii* suspended cell system was thus obtained; and
(4) on days 1 to 31 of the culture, the *Siraitia grosvenorii* suspended cells were extracted every other day to detect the **content** of mogroside V and **mogroside II.**

### Comparison of contents of mogroside V

### 1. Experimental method

Weighing 1.5 g *Siraitia grosvenorii* dry weight cells, adding 60% ethanol at a feed-to-liquid ratio of 15:1, ultrasonically extracting for 40 min, extracting for 2 h at 90°C in a slightly boiling state, repeating the extraction for 3 times, and combining the extracts to concentrate them to 5 mL in a rotary evaporator. The concentrate was filtered through a 0.22 µm filtration membrane, and then qualitative and quantitative analysis was performed using a high-performance liquid chromatograph.

Chromatographic conditions: The chromatographic column was an inverted C18 column, 4.6 mm × 250 mm; the mobile phase was water-acetonitrile at a ratio of 78:22, the flow rate was 1.0 mL/min; and the detection wavelength was 203 nm.

### 2. Results

The effect of different treatment conditions on the yield of mogroside V per unit time is shown in Table 1.

**Table 1. The effect of different treatment conditions on the yield of mogroside V per unit time (n=5; x ±s g/L)**

| | **Example 1** | **Comparative Example 1** | **Comparative Example 2** | **Comparative Example 3** | **Comparative Example 4** | **Comparativ e Example 5** |
|---|---|---|---|---|---|---|
| 1 d | 31.1±0.8 | 31.4±0.6 | 32.6±0.5 | 31.5±0.5 | 31.1 ±0.8 | 30.0± 1.0 |
| 3 d | 32.6±12 | 31.9±1.4 | 33.0±1.6 | 31.8±2.1 | 32.6±0.9 | 31.6±0.6 |
| 5 d | 33.6+1.1 | 32.9±1.2 | 33.2±1.4 | 32.8±1.8 | 33.2±1.1 | 32.2±0.8 |
| 7 d | 34.2+1.4 | 33.2±1.1 | 34.2±1.8 | 33.0±1.6 | 33.5±1.5 | 33.6±1.2 |
| 9 d | 43.5±2.1 | 33.8±1.3 | 35.6±1.5 | 33.6±1.4 | 39.5±2.0 | 39.8±1.5 |
| 11 d | 52.6±1.8 | 34.2±1.6 | 36.8±1.6 | 34.0±1.2 | 46.5±1.8 | 45.5±1.7 |
| 13 d | 64.8±2.0 | 34.7±1.5 | 41.5±2.0 | 34.5±2.2 | 51.6±1.8 | 50.5±2.2 |
| 15 d | 73.8±2.8 | 35.2±2.2 | 47.5+2.6 | 36.8±1.6 | 53.6±1.5 | 56.4±2.4 |
| 17 d | 79.9±2.6 | 36.2±2.1 | 53.4±2.2 | 43.5±1.8 | 54.5±1.6 | 60.8±2.8 |
| 19 d | 85.2±2.5 | 37.8±2.4 | 60.8±2.1 | 49.5±2.0 | 55.7±1.8 | 67.5+2.5 |
| 21 d | 79.5±2.6 | 39.4±2.5 | 67.2+2.3 | 55.4±2.2 | 56.8+2.0 | 70.7±3.1 |
| 23 d | 78.6±2.8 | 52.6+3.0 | 72.0±2.8 | 61.8±2.6 | 58.2±2.2 | 69.2±3.2 |
| 25 d | 76.5±2.5 | 64.8±2.8 | 75.6+2.6 | 68.5±2.5 | 55.2±2.4 | 65.8±3.0 |
| 27 d | 74.2±2.6 | 73.8±2.5 | 77.2+2.8 | 71.8±3.0 | 51.4±2.5 | 63.6±2.8 |
| 29 d | 72.1+3.1 | 75.9±2.9 | 79.0±2.5 | 72.2±2.7 | 46.5±2.4 | 59.4±3.2 |
| 31 d | 68.2±2.8 | 76.2±2.6 | 78.8±2.5 | 72.0±2.8 | 41.2±2.5 | 55.6±2.8 |

The effect of different treatment conditions on the peak yield (the maximum yield) of mogroside V is shown in Table 2.

**Table 2. The effect of different treatment conditions on the peak yield (the maximum yield) of mogroside V (n=5; x ±s; g/L)**

| | Peak yield (maximum yield) of mogroside V |
|---|---|
| **Example 1** | 85.2±2.5 |
| **Example 2** | 84.9±1.8 |
| **Example 3** | 86.7±2.2 |
| **Example 4** | 85.1±3.0 |
| **Comparative Example 1** | 76.2±2.6 |
| **Comparative Example 2** | 79.0±2.5 |
| **Comparative Example 3** | 72.2 ±2.7 |
| **Comparative Example 4** | 58.2±2.2 |
| **Comparative Example 5** | 70.7±3.1 |

### Comparison of contents of mogroside II

### 1. Experimental method

Weighing 1.5 g *Siraitia grosvenorii* dry weight cells, adding 60% ethanol at a feed to liquid ratio of 15:1, ultrasonically extracting for 40 min, extracting for 2 h at 90°C in a slightly boiling state, repeating the extraction for 3 times, and combining the extracts to concentrate them to 5 mL in a rotary evaporator. The concentrate was filtered through a 0.22 µm filtration membrane, and then qualitative and quantitative analysis was performed using a high-performance liquid chromatograph.

Chromatographic conditions: The chromatographic column was a ZORBAX SBC C18 column, 4.6 mm × 150 mm; the mobile phase was water-acetonitrile gradient elution (0-25 min, 13.5% to 35% acetonitrile), the flow rate was 0.8 mL/min; and the detection wavelength was 203 nm.

### 2. Results

The effect of different treatment conditions on the content of **mogroside II** at the peak of mogroside V is shown in Table 3.

**Table 3. The effect of different treatment conditions on the content of mogroside II at the peak of mogroside V (n=5; x ±s; g/L)**

| | **Content of mogroside II** |
|---|---|
| **Example 1** | 17.2±2.1 |
| **Example 2** | 17.6±2.0 |
| **Example 3** | 18.5±1.4 |
| **Example 4** | 18.3±2.6 |
| **Comparative Example 1** | 42.6±4.3 |
| **Comparative Example 2** | 39.8±3.8 |
| **Comparative Example 3** | 40.2±2.2 |
| **Comparative Example 4** | 32.8±2.6 |
| **Comparative Example 5** | 30.4±1.6 |

### Conclusion

The results of comparison of the contents of mogroside V (Tables 1 and 2) show that the contents of mogroside V in Examples 1-4 were significantly higher than those of Comparative Examples 1-5, with the peak values at 85.2 g/L, 84.9±1.8 g/L, 86.7±2.2 g/L and 85.1±3.0 g/L, respectively, indicating that the addition of the yeast inducer and methyl jasmonate to the *Siraitia grosvenorii* suspended cell culture system could effectively increase the yield of mogroside V, with the technical effect obviously superior to the prior art. Besides, in comparison with Comparative Examples 1-5, the synthesis rate of mogroside V in Example 1 was significantly increased, with the content of mogroside V higher than 70 g/L on day 15 of the culture and reaching the peak value on day 19 of the culture. The results show that the addition of the yeast inducer and methyl jasmonate in the *Siraitia grosvenorii* suspended cell culture system could effectively increase the synthesis rate of mogroside V by the *Siraitia grosvenorii* suspended cells, with the technical effect obviously superior to the prior art.

The results of comparison of the contents of mogroside II (Table 3) show that the contents of mogroside II in Examples 1-5 were significantly reduced compared with Comparative Examples 1-5, indicating that the addition of the yeast inducer and methyl jasmonate to the *Siraitia grosvenorii* suspended cell culture system could effectively reduce the content of the bitter component mogroside II, with the technical effect obviously superior to the prior art.

Although the present invention has been described in detail by using general descriptions, specific embodiments and tests, some modifications or improvements can be made based on the present invention, which will be obvious to those skilled in the art. Therefore, these modifications or improvements made without departing from the spirit of the present invention shall all fall within the scope of protection of the present invention.

### Industrial practicality

The present invention provides a method for increasing the content of mogroside V in *Siraitia grosvenorii* suspended cells. In the method, 10-200 mg/L of a yeast inducer and 50-260 mg/L of methyl jasmonate are respectively added on days 5 to 11 of the culture cycle of *Siraitia grosvenorii* suspended cells, thereby increasing the synthesis rate and yield of mogroside V, and also reducing the amount of mogroside II secreted. The method of the present invention, artificially regulating the production process of *Siraitia grosvenorii* cells and mogroside V, is beneficial for the market-oriented and large-scale production and quality control management of mogroside V, and has good economic value and broad application prospects.

## Claims

1. **A method for increasing content of mogroside V in *Siraitia grosvenorii*** suspended cells, **characterized in that** a yeast inducer and methyl jasmonate are added during culture of mature *Siraitia grosvenorii* suspended cells in a primary *Siraitia grosvenorii* suspended cell system.

2. **The method according to claim 1, characterized in that** during the culture of the mature *Siraitia grosvenorii* suspended cells in the primary *Siraitia grosvenorii* suspended cell system, 10-50 mg/L of the yeast inducer and 50-100 µmol/L of methyl jasmonate are added on days 5 to 7, 100-200 mg/L of the yeast inducer and 120-260 µmol/L of methyl jasmonate are added on days 9 to 11, and the mature *Siraitia grosvenorii* suspended cells are harvested on days 17 to 25.

3. The method according to claim 1, **characterized in that** during the culture of the mature *Siraitia grosvenorii* suspended cells in the primary *Siraitia grosvenorii* suspended cell system, 50 mg/L of the yeast inducer and 100 µmol/L of methyl jasmonate are added on day 7, 180 mg/L of the yeast inducer and 220 µmol/L of methyl jasmonate are added on day 11, and the mature *Siraitia grosvenorii* suspended cells are harvested on day 19.

4. **The method according to any one of claims 1-3, characterized in that** the primary *Siraitia grosvenorii* suspended cell system is obtained by the following steps:
(1) taking mature *Siraitia grosvenorii* embryos, subculturing on an MS semi-solid medium containing 30 g/L of sucrose, 1.0 mg/L of 6-benzylamino adenine, 0.5 mg/L of naphthaleneacetic acid, 100 mg/L of inositol and 4.6 g/L of agar in a pH range of 5.5-6.0, and selecting fresh embryogenic calli that are successively subcultured 3-5 times, grow vigorously, and have loose texture and a stable and uniform state; and
(2) the fresh embryogenic calli obtained in the step (1) are transferred at an inoculum amount of 100 g/L to an MS liquid medium containing 30 g/L of sucrose, 1.0 mg/L of 6-benzylamino adenine, 0.5 mg/L of naphthaleneacetic acid and 100 mg/L of inositol in a pH range of 5.5-6.5, and cultured in a shaker at a rotation speed of 150 r/min, a culture temperature of (25±2)°C, a light intensity of 4000 lux and an illumination time of 12 h/d, so that the primary *Siraitia grosvenorii* suspended cell system is obtained.

5. The method according to claim 4, **characterized in that** the subculture is performed in the step (1) for 4 times.

6. A method for preparing the mogroside V, **characterized in that** the mature *Siraitia grosvenorii* suspended cells prepared by the method according to any one of claims 1-3 and 5 are further extracted and isolated to prepare the mogroside V.

7. A method for preparing the mogroside V, **characterized in that** the mature *Siraitia grosvenorii* suspended cells prepared according to claim 4 are further extracted and isolated to prepare the mogroside V.

## Patentansprüche

1. Verfahren zur Erhöhung des Gehalts von Mogrosid V in suspendierten Zellen von *Siraitia grosvenorii,* **dadurch gekennzeichnet, dass** ein Hefeinduktor und Methyljasmonat während der Kultivierung von reifen suspendierten Zellen von *Siraitia grosvenorii* zu einem System von suspendierten primären Zellen von *Siraitia grosvenorii* hinzugegeben werden.

2. Verfahren gemäß Anspruch 1, **dadurch gekennzeichnet, dass** während der Kultivierung der reifen suspendierten Zellen von *Siraitia grosvenorii* in dem System von suspendierten primären Zellen von *Siraitia grosvenorii* an den Tagen 5 bis 7 10-50 mg/L des Hefeinduktors und 50-100 µmol/L von Methyljasmonat hinzugeben werden, an den Tagen 9-11 100-200 mg/L des Hefeinduktors und 120-260 µmol/L von Methyljasmonat hinzugegeben werden, und die reifen suspendierten Zellen von *Siraitia grosvenorii* an den Tagen 17 bis 25 geerntet werden.

3. Verfahren gemäß Anspruch 1, **dadurch gekennzeichnet, dass** während der Kultivierung der reifen suspendierten Zellen von *Siraitia grosvenorii* in dem System von suspendierten primären Zellen von *Siraitia grosvenori* an dem Tag 7 50 mg/l des Hefeinduktors und 100 µmol/L von Methyljasmonat hinzugeben werden, an dem Tag 11 180 mg/L des Hefeinduktors und 220 µmol/L von Methyljasmonat hinzugegeben werden, und die reifen suspendierten Zellen von *Siraitia grosvenorii* an dem Tag 19 geerntet werden.

4. Verfahren gemäß einem der Ansprüche 1-3, **dadurch gekennzeichnet, dass** das System von suspendierten primären Zellen von *Siraitia grosvenorii* durch die folgenden Schritte erhalten wird:
(1) Entnahme von reifen Embryos von *Siraitia grosvenorii,* Subkultivierung auf einem halbfesten MS-Medium, das 30 g/L Saccharose, 1,0 mg/L 6-Benzylaminoadenin, 0,5 mg/L Naphthalinessigsäure, 100 mg/L Inositol und 4,6 g/L Agar in einem pH-Bereich von 5,5-6,0 enthält, und Selektieren von frischen embryogenen Kalli, die sukzessive 3-5 mal subkultiviert werden, kräftig wachsen, und eine lockere Textur sowie einen stabilen und einheitlichen Zustand aufweisen; und
(2) Übertragen der in Schritt (1) erhaltenen frischen embryogenen Kalli mit einer Inokulummenge von 100 g/L in ein flüssiges MS-Medium, das 30 g/L Saccharose, 1,0 mg/L 6-Benzylaminoadenin, 0,5 mg/L Naphthalinessigsäure und 100 mg/L Inositol in einem pH-Bereich von 5,5-6,5 enthält, und Kultivieren in einem Schüttler bei einer Rotationsgeschwindigkeit von 150 U/min, einer Kultivierungstemperatur von (25 ± 2)°C, einer Lichtintensität von 4000 Lux und einer Beleuchtungszeit von 12 h/d, so dass das System von suspendierten primären Zellen von *Siraitia grosvenorii* erhalten wird.

5. Verfahren gemäß Anspruch 4, **dadurch gekennzeichnet, dass** die Subkultivierung in Schritt (1) viermal durchgeführt wird.

6. Verfahren zur Herstellung von Mogrosid V, **dadurch gekennzeichnet, dass** die reifen suspendierten Zellen von *Siraitia grosvenorii,* die durch das Verfahren gemäß einem der Ansprüche 1-3 und 5 hergestellt wurden, weiter extrahiert und isoliert werden, um das Mogrosid V herzustellen.

7. Verfahren zur Herstellung von Mogrosid V, **dadurch gekennzeichnet, dass** die reifen suspendierten Zellen von *Siraitia grosvenorii,* die gemäß Anspruch 4 hergestellt wurden, weiter extrahiert und isoliert werden, um das Mogrosid V herzustellen.

## Revendications

1. Procédé pour augmenter la teneur en mogroside V dans des cellules de *Siraitia grosvenorii* en suspension, **caractérisé en ce qu'**un inducteur de levure et du jasmonate de méthyle sont ajoutés pendant la culture de cellules matures de *Siraitia grosvenorii* en suspension dans un système de cellules primaires de *Siraitia grosvenorii* en suspension.

2. Procédé selon la revendication 1, **caractérisé en ce que** lors de la culture des cellules matures de *Siraitia grosvenorii* en suspension dans le système de cellules primaires de *Siraitia grosvenorii* en suspension, 10 à 50 mg/l de l'inducteur de levure et 50 à 100 µmole/l de jasmonate de méthyle sont ajoutés aux jours 5 à 7, 100 à 200 mg/l de l'inducteur de levure et 120 à 260 µmole/l de jasmonate de méthyle sont ajoutés aux jours 9 à 11, et les cellules matures de *Siraitia grosvenorii* en suspension sont récoltées aux jours 17 à 25.

3. Procédé selon la revendication 1, **caractérisé en ce que** lors de la culture des cellules matures de *Siraitia grosvenorii* en suspension dans le système de cellules primaires de *Siraitia grosvenorii* en suspension, 50 mg/l de l'inducteur de levure et 100 µmole/l de jasmonate de méthyle sont ajoutés au jour 7, 180 mg/l de l'inducteur de levure et 220 µmole/l de jasmonate de méthyle sont ajoutés au jour 11, et les cellules matures de *Siraitia grosvenorii* en suspension sont récoltées au jour 19.

4. Procédé selon l'une quelconque des revendications 1 à 3, **caractérisé en ce que** le système de cellules primaires de *Siraitia grosvenorii* en suspension est obtenu par les étapes suivantes :
(1) prélever des embryons matures de *Siraitia grosvenorii,* repiquer sur un milieu MS semi-solide contenant 30 g/l de saccharose, 1,0 mg/l de 6-benzylamino adénine, 0,5 mg/l d'acide naphtalène acétique, 100 mg/l d'inositol et 4,6 g/l d'agar dans une plage de pH de 5,5 à 6,0, et sélectionner des cals embryonnaires frais qui sont successivement repiqués 3 à 5 fois, croissent vigoureusement, et ont une texture lâche et un état stable et uniforme ; et
(2) les cals embryonnaires frais obtenus à l'étape (1) sont transférés à une quantité d'inoculum de 100 g/l dans un milieu MS liquide contenant 30 g/l de saccharose, 1,0 mg/l de 6-benzylamino adénine, 0,5 mg/l d'acide naphtalène acétique et 100 mg/l d'inositol dans une plage de pH de 5,5 à 6,5, et cultivés dans un agitateur à une vitesse de rotation de 150 tr/min, une température de culture de (25 ± 2) °C, une intensité de lumière de 4 000 lux et un temps d'éclairage de 12 h/j, de sorte que le système de cellules primaires de *Siraitia grosvenorii* en suspension est obtenu.

5. Procédé selon la revendication 4, **caractérisé en ce que** la sous-culture est effectuée 4 fois à l'étape (1).

6. Procédé de préparation du mogroside V, **caractérisé en ce que** les cellules matures de *Siraitia grosvenorii* en suspension préparées par le procédé selon l'une quelconque des revendications 1 à 3 et 5 sont en outre extraites et isolées pour préparer le mogroside V.

7. Procédé de préparation du mogroside V, **caractérisé en ce que** les cellules matures de *Siraitia grosvenorii* en suspension préparées selon la revendication 4 sont en outre extraites et isolées pour préparer le mogroside V.
